# EUROPEAN PATENT SPECIFICATION

(11) **EP 1 301 783 B1**
(45) Date of publication and mention of the grant of the patent: **15.09.2004**
(21) Application number: 01947699.3
(22) Date of filing: 17.07.2001
(51) Int. Cl.: G01N 33/487

(54) **TEST MEMBER**
TESTVORRICHTUNG
ELEMENT D'ESSAI

(30) Priority: 20.07.2000 GB 0017738
(43) Date of publication of application: 16.04.2003
(73) Proprietor: HYPOGUARD LIMITED, Woodbridge Suffolk IP12 1PE (GB)
(72) Inventor: BLACK, Murdo M., Forfar, Angus, DD8 2AZ (GB)
(74) Representative: Gemmell, Peter Alan, Dr.
(86) International application number: PCT/GB2001/003208
(87) International publication number: WO 2002/008750

(56) References cited:
- EP-A- 0 159 727
- EP-A- 0 474 145
- EP-A- 0 785 433
- WO-A-94/10558
- WO-A-99/13101
- GB-A- 2 328 023

## Description

### 1. Field of the Invention

The present invention relates to a test member for measuring the concentration of an analyte in a fluid sample, notably to a test strip for analysing blood glucose or other analytes in bodily fluids. The invention also provides a test device which includes a stack of the test members.

### 2. Background of the Invention

Diabetics regularly need to test samples of their blood to determine the level of blood glucose. The results of such tests may be used to determine levels of medication needed to treat the diabetes at the time. In one known type of system, disposable sensors are used to test the blood. The sensors typically take the form of test strips which are provided with a reagent material that will react with blood glucose to produce an electrical signal. Conductive tracks on the test strip relay the electrical signal to a meter which displays the result. After a sample of blood has been applied to the test strip and the measurement has been taken, the test strip is disposed of. In order to couple the conductive tracks on a test strip with the meter, the test strip needs to be inserted into a sensor holder prior to the start of testing. The sensor holder has corresponding electrodes which are brought into electrical contact with the conductive tracks of the test strip. An example of an amperometric test strip which requires low sample volumes is described in WO 99/13101.

A reduction in minimum sample size is achieved by means of a dielectric coating impregnated into peripheral regions by one or more hydrophilic mesh layers, thereby reducing sample dead volume.

Alternatively, the reagent in the test strip may undergo a visible colour change, the magnitude of which is used to determine the analyte concentration in the applied fluid. An example of such a test strip is described in EP 0 159 727.

It is known to provide a stack of disposable circular test elements in a cylindrical housing, the stack being urged towards a test station by a spring to form a liquid-proof seal, for example as described in WO 94/10558.

A problem with providing disposable test members in a stack is that the working area to which the fluid sample will be applied can become scuffed, particularly when a compressive force is applied to the stack by a spring.

It is an object of the present invention to provide an improved test member suitable for use in test devices that employ test members in a stack.

### SUMMARY OF THE INVENTION

According to an aspect of the present invention there is provided a test device as specified in claim 1.

By making at least a part of the non-working area thicker than the working area, scuffing or abrasion of the working area in a stack can be reduced. Moreover, if a compressive load is applied to a stack of the test members, this may be spread out over a greater area, thereby reducing the possibility of compressive damage to the working area. The difference in thickness is preferably from 1 to 20 µm, notably from 5 to 10 µm.

Another aspect of the invention provides a test member suitable for use in the test device, as specified in claim 13.

The test member may be of any desired shape for a particular application; however, typically the test member will be an elongate test strip. For convenience hereinafter, the invention will be described with reference to such a test strip. However, it is to be understood that the invention is not limited to this embodiment.

The reagent reacts with the analyte to produce an electrical signal which is measured and displayed by a meter. The working area has electrodes which are electrically connected to electrode tracks in the non-working area, and at least part of the tracks are exposed for connection to electrodes of a meter.

To build up the working area, a plurality of layers are sequentially applied to the base layer, for example by screen printing, typically with curing or drying steps between the application steps. The layers which are printed typically comprise electrode patterns, a reagent layer, and a mesh layer (for spreading out an applied fluid). As a result of the application of these layers, the working area of a conventional electrochemical test strip is typically about 100 µm thicker than the non-working area, which contains the electrode tracks and, typically, a dielectric layer. A stack of 100 test strips will therefore be about 10 mm thicker in the working area than in the non-working area. In a test strip in accordance with the present invention, at least a part of the non-working area may be made thicker by any suitable means. Suitable means include, for example: a printed relief ink; an applied pad or tape; embossing of the base layer or an intermediate layer; or an extension of the mesh layer from the working area.

The test members may be held under a compressive load by spring means.

### BRIEF DESCRIPTION OF THE DRAWINGS

The invention will now be further described, by way of example, with reference to the following drawing in which:

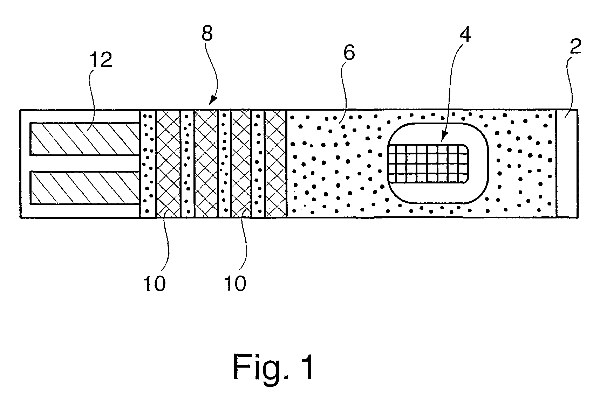

Figure 1 is a top plan view of a test strip in accordance with the present invention.

### DETAILED DESCRIPTION

The exemplified test strip comprises a planar base member 2, in this example of poly(butylene terephthalate) (PBT) (Valox® FR-1 from GE Plastics). The strip is 30 mm x 5.5 mm, and 0.5 mm thick. A working area 4 is of conventional construction, comprising a plurality of electrodes, a reagent layer in intimate contact with the electrodes, and a mesh layer for spreading out a drop of fluid to be received on the working area. Electrode tracks 12, for example of carbon, in the non-working area 8 of the test strip are connected to the electrodes in the working area 4 in known manner. Also in known manner, a dielectric layer 6 is printed around the working area 4 so as to overlie a portion of the electrode tracks 12, leaving just the ends of the tracks exposed for connection to corresponding electrodes on a meter. The layers are applied to the base member as inks, by screen printing. Each ink layer is about 10 to 20 µm thick, and the mesh is about 59 to 67 µm thick. The working area 4 has a total thickness which is about 100 µm thicker than the non-working area 8 up to the dielectric layer 6.

To increase the thickness of parts of the non-working area, a high relief ink 10 has been printed in four strips. The high relief ink has a dried thickness such that the total thickness of the non-working area to which the high relief ink 10 has been applied is slightly greater than the total thickness of the test strip in the working area 4. Thus, when a stack of such test strips is formed, and a compressive load is applied to the stack by a spring, the working area 4 will not bear all the compressive load. If the test strips are used in a device which requires one strip to be slid out before being used to test analyte concentration in a fluid, scuffing of the test area will be reduced compared to a conventional test strip in which the working area stands proud of the non-working area.

Although the invention has been illustrated with reference to the use of a high relief ink printed in strips, it will be understood that it is not limited to this embodiment. The ink could be printed as a continuous block, and it could entirely surround the working area if desired. Instead of, or in addition to, the high relief ink, other means could also be provided to increase the thickness of the non-working area, for example: an applied pad or tape; embossing of the base layer or an intermediate layer; or an extension of the mesh layer from the working area into the non-working area.

## Claims

1. A test device for testing of analyte concentration in a fluid to be applied thereto, the device including a plurality of test members arranged in at least one stack, each of said test members carrying printed reagent means for producing an electrical signal in response to the concentration of analyte in an applied fluid; wherein each of the said test members comprises a base member (2) having on one surface a working area (4) to which the fluid is to be applied, containing the said reagent means and a plurality of electrodes, and having on the same surface a non-working area (8) adjacent to the working area (4), the said non-working area (8) comprising a plurality of electrode tracks (12) on the base member (2) which are electrically connected to the electrodes in the working area (4), and a dielectric layer (6) which overlies a portion of the electrode tracks (12), leaving the ends of the tracks (12) exposed for connection to corresponding electrodes on the meter;
**characterised in that** the total thickness of each test member in at least a portion (10) of the non-working area (8) is greater than the total thickness of each test member in the working area (4).

2. A test device as claimed in claim 1, wherein the at least one stack of test members is held under a compressive load by spring means.

3. A test device as claimed in any preceding claim, wherein for each test member the total thickness in at least a part (10) of the non-working area (8) is from 1 to 20 µm greater than the total thickness in the working area (4).

4. A test device as claimed in claim 3, wherein for each test member the total thickness in at least a part (10) of the non-working area (8) is from 5 to 10 µm greater than the total thickness in the working area (4).

5. A test device as claimed in any one of the preceding claims wherein the said at least a part (10) of the non-working area (8) includes a printed ink layer.

6. A test device as claimed in any one of the preceding claims wherein the said at least a part (10) of the non-working area (8) includes an adhered tape or pad.

7. A test device as claimed in any one of the preceding claims wherein the said at least a part (10) of the non-working area (8) includes an embossed structure.

8. A test device as claimed in any one of the preceding claims wherein the said at least a part (10) of the non-working area (8) includes a mesh layer.

9. A test device as claimed in claim 8, wherein the said mesh layer is continuous with a mesh layer in the working area (4).

10. A test device as claimed in any one of the preceding claims, wherein the test members are elongate test strips.

11. A test device as claimed in any one of the preceding claims, suitable for use in testing for the concentration of glucose in blood.

12. A test device as claimed in any one of the preceding claims, wherein the reagent means on each test member comprises a screen-printed layer.

13. A test member for use in the device of claim 1, the test member carrying printed reagent means for producing an electrical signal in response to the concentration of analyte in an applied fluid; wherein the test member comprises a base member (2) having on one surface a working area (4) to which the fluid is to be applied, containing the said reagent means and a plurality of electrodes, and having on the same surface a non-working area (8) adjacent to the working area (4), the said non-working area (8) comprising a plurality of electrode tracks (12) on the base member which are electrically connected to the electrodes in the working area (4), and a dielectric layer (6) which overlies a portion of the electrode tracks (12), leaving the ends of the tracks (12) exposed for connection to corresponding electrodes on a meter;
**characterised in that** the total thickness of the test member in at least a portion (10) of the non-working area (8) is greater than the total thickness of the test member in the working area (4).

14. A test member as claimed in claim 13, wherein the total thickness in at least a part (10) of the non-working area (8) is from 1 to 20 µm greater than the total thickness in the working area (4).

15. A test device as claimed in claim 13, wherein the total thickness in at least a part (10) of the non-working area (8) is from 5 to 10 µm greater than the total thickness in the working area (4).

## Patentansprüche

1. Testanordnung zum Testen einer Analytkonzentration in einem darauf aufzubringenden Fluid, die eine Vielzahl von Testvorrichtungen aufweist, die in mindestens einem Stapel angeordnet sind, wobei jede Testvorrichtung aufgedruckte Reagenzmittel trägt, um in Reaktion auf die Analytkonzentration in einem aufgebrachten Fluid ein elektrisches Signal zu erzeugen; wobei jedes der Testvorrichtungen ein Basiselement (2) umfasst, das auf einer Oberfläche eine Arbeitszone (4) aufweist, auf die das Fluid aufgebracht werden muss, und die Reagenzmittel und eine Vielzahl von Elektroden aufweist, und das auf der selben Oberfläche eine Nicht-Arbeitszone (8) benachbart zur Arbeitszone (4) aufweist, wobei die Nicht-Arbeitszone (8) eine Vielzahl von Elektrodenbahnen (12) auf dem Basiselement (2) umfasst, die elektrisch mit den Elektroden in der Arbeitszone (4) verbunden sind, und einen dielektrischen Layer (6) aufweist, der einen Bereich der Elektrodenbahnen (12) überdeckt, wodurch die Enden der Bahnen (12) zur Verbindung mit korrespondierenden Elektroden des Messgerätes freibleiben,
**dadurch gekennzeichnet,**
**dass** die Gesamtdicke jeder Testvorrichtung in mindestens einem Bereich (10) der Nicht-Arbeitszone (18) größer ist als die Gesamtdicke jeder Testvorrichtung in der Arbeitszone (4).

2. Testanordnung nach Anspruch 1, in der der mindestens eine Stapel von Testvorrichtungen durch Federmittel unter einer Druckkraft gehalten wird.

3. Testanordung nach einem der vorhergehenden Ansprüche, in der die Gesamtdicke für jede Testvorrichtung in mindestens einem Bereich (10) der Nicht-Arbeitszone (8) 1 bis 20 µm größer ist, als die Gesamtdicke in der Arbeitszone (4).

4. Testanordnung nach Anspruch 3, in der für jede Testvorrichtung die Gesamtdicke in mindestens einem Bereich (10) der Nicht-Arbeitszone (8) 5 bis 10 µm größer ist als die Gesamtdicke in der Arbeitszone (4).

5. Testanordnung nach einem der vorhergehenden Ansprüche, in der mindestens ein Bereich (10) der Nicht-Arbeitszone (8) einen aufgedruckten Layer aus einer graphischen Farbe umfasst.

6. Testanordnung nach einem der vorhergehenden Ansprüche, in der mindestens ein Bereich (10) der Nicht-Arbeitszone (8) ein anhaftendes Band oder Pad umfasst.

7. Testanordnung nach einem der vorhergehenden Ansprüche, in der mindestens ein Bereich (10) der Nicht-Arbeitszone (8) eingeprägte Strukturen umfasst.

8. Testanordnung nach einem der vorhergehenden Ansprüche, in der mindestens ein Bereich (10) der Nicht-Arbeitszone (8) einen Gitterlayer umfasst.

9. Testanordnung nach Anspruch 8, in der der Gitterlayer einstückig mit einem Gitterlayer in der Arbeitszone (4) ist.

10. Testanordnung nach einem der vorhergehenden Ansprüche, in der die Testvorrichtungen längliche Teststreifen sind.

11. Testanordnung nach einem der vorhergehenden Ansprüche, geeignet zur Verwendung beim Bestimmen der Blutzuckerkonzentration.

12. Testanordnung nach einem der vorhergehenden Ansprüche, in der die Reagenzmittel auf jeder Testvorrichtung einen siebbedruckten Layer umfassen.

13. Testvorrichtung zur Verwendung in der Anordnung nach Anspruch 1, die aufgedruckte Reagenzmittel zur Erzeugung eines elektrischen Signals in Reaktion auf die Analytkonzentration in einem aufgebrachten Fluid trägt und die ein Basiselement (2) umfasst, das auf einer Oberfläche eine Arbeitszone (4) aufweist, auf die das Fluid aufgebracht werden muss, die Reagenzmittel und die Vielzahl von Elektroden beinhaltet und auf derselben Oberfläche eine Nicht-Arbeitszone (8) benachbart zu der Arbeitszone (4) aufweist, wobei die Nicht-Arbeitszone (8) eine Vielzahl von Elektrodenbahnen (12) auf dem Basiselement, die elektrisch mit den Elektroden in der Arbeitszone (4) verbunden sind und einen dielektrischen Layer (6) umfasst, der einen Bereich der Elektrodenbahnen (12) überdeckt, wobei die Enden der Bahnen (12) zur Verbindung mit den entsprechenden Elektroden am Messgerät freibleiben,
**dadurch gekennzeichnet,**
**dass** die Gesamtdicke der Testvorrichtung in mindestens einem Bereich (10) der Nicht-Arbeitszone (8) größer ist als die Gesamtdicke der Testvorrichtung in der Arbeitszone (4).

14. Testvorrichtung nach Anspruch 13, in der die Gesamtdicke in mindestens einem Bereich (10) der Nicht-Arbeitszone (8) 1 bis 20 µm größer ist als die Gesamtdicke in der Arbeitszone (4).

15. Testanordnung nach Anspruch 13, in der die Gesamtdicke in mindestens einem Bereich (10) der Nicht-Arbeitszone (8) 5 bis 10 µm größer ist als die Gesamtdicke in der Arbeitszone (4).

## Revendications

1. Dispositif d'essai pour tester la concentration de substance à analyser dans un fluide à appliquer dessus, le dispositif comprenant une pluralité d'éléments d'essai agencés dans au moins une pile, chacun desdits éléments d'essai ayant des moyens de réactif imprimés pour produire un signal électrique en réponse à la concentration de substance à analyser dans un fluide appliqué ; dans lequel chacun desdits éléments d'essai comprend un élément de base (2) ayant sur une surface une zone de travail (4) à laquelle le fluide doit être appliqué, contenant lesdits moyens de réactif et une pluralité d'électrodes et ayant sur la même surface une zone de non-travail (8) adjacente à la zone de travail (4), ladite zone de non-travail (8) comprenant une pluralité de pistes d'électrode sur l'élément de base (2) qui sont électriquement connectées aux électrodes dans la zone de travail (4), et une couche diélectrique (6) qui recouvre une partie des pistes d'électrode (12), laissant les extrémités des pistes (12) exposées pour être connectées aux électrodes correspondantes sur le compteur ;
caractérisé en ce l'épaisseur totale de chaque élément de test dans au moins une partie (10) de la zone de non-travail (8) est supérieure à l'épaisseur totale de chaque élément de test dans la zone de travail (4).

2. Dispositif d'essai selon la revendication 1, dans lequel au moins une pile d'éléments d'essai est maintenue sous une charge compressive par des moyens de ressorts.

3. Dispositif d'essai selon l'une quelconque des revendications précédentes, dans lequel pour chacun des éléments d'essai l'épaisseur totale dans au moins une partie (10) de la zone de non-travail (8) est de 1 à 20 µm supérieure à l'épaisseur totale dans la zone de travail (4).

4. Dispositif d'essai selon la revendication 3, dans lequel pour chaque élément d'essai l'épaisseur totale dans au moins une partie (10) de la zone de non-travail (8) est de 5 à 10 µm supérieure à celle de l'épaisseur totale dans la zone de travail (4).

5. Dispositif d'essai selon l'une quelconque des revendications précédentes, dans lequel ladite au moins une partie (10) de la zone de non-travail (8) comprend une couche d'encre imprimée.

6. Dispositif d'essai selon l'une quelconque des revendications précédentes, dans lequel ladite au moins une partie (10) de la zone de non-travail (8) comprend une bande ou une pastille adhérée.

7. Dispositif d'essai selon l'une quelconque des revendications précédentes, dans lequel ladite au moins une partie (10) de la zone de non-travail (8) comprend une structure estampée.

8. Dispositif d'essai selon l'une quelconque des revendications précédentes, dans lequel ladite au moins une partie (10) de la zone de non-travail (8) comprend une couche de maillage.

9. Dispositif d'essai selon la revendication 8, dans lequel la couche de maillage est continue avec une couche de maillage dans la zone de travail (4).

10. Dispositif d'essai selon l'une quelconque des revendications précédentes, dans lequel les éléments d'essai sont des bandes d'essai allongées.

11. Dispositif d'essai selon l'une quelconque des revendications précédentes, adapté pour être utilisé dans les essais de concentration du glucose contenu dans le sang.

12. Dispositif d'essai selon l'une quelconque des revendications précédentes, dans lequel les moyens de réactif sur chaque élément d'essai comprennent une couche sérigraphiée.

13. Élément d'essai à utiliser avec le dispositif d'essai selon la revendication 1, l'élément d'essai ayant des moyens de réactif imprimés pour produire un signal électrique en réponse à la concentration de substance à analyser dans un fluide appliqué ; dans lequel l'élément d'essai comprend un élément de base (2) ayant sur une surface une zone de travail (4) à laquelle le fluide doit être appliqué, contenant lesdits moyens de réactif et une pluralité d'électrodes et ayant sur la même surface une zone de non-travail (8) adjacente à la zone de travail (4), ladite zone de non-travail (8) comprenant une pluralité de pistes d'électrode (12) sur l'élément de base qui sont électriquement connectées aux électrodes dans la zone de travail (4), et une couche diélectrique (6) qui recouvre une partie des pistes d'électrode (12), laissant les extrémités des pistes (12) exposées pour être connectées aux électrodes correspondantes sur un compteur ;
caractérisé en ce l'épaisseur totale de chaque élément de test dans au moins une partie (10) de la zone de non-travail (8) est supérieure à l'épaisseur totale de chaque élément de test dans la zone de travail (4).

14. Élément d'essai selon la revendication 13, dans lequel l'épaisseur totale dans au moins une partie (10) de la zone de non-travail (8) est de 1 à 20 µm supérieure à l'épaisseur totale dans la zone de travail (4).

15. Élément d'essai selon la revendication 13, dans lequel l'épaisseur totale dans au moins une partie (10) de la zone de non-travail (8) est de 5 à 10 µm supérieure à celle de l'épaisseur totale dans la zone de travail (4).
